# EUROPEAN PATENT APPLICATION

(11) **EP 4 166 944 A1**
(43) Date of publication of application: **19.04.2023**
(21) Application number: 21820957.5
(22) Date of filing: 10.06.2021
(51) Int. Cl.: G01N 33/48, G01N 33/50

(54) **SUPERABSORBENT POLYMER FOR CLASSIFICATION OF PARTICLES AND CLASSIFICATION METHOD USING THE SAME**

(30) Priority: 12.06.2020 JP 2020102227
(71) Applicant: Tokushima University, Tokushima-shi, Tokushima 770-8501 (JP)
(72) Inventor: ABE, Hideharu, Tokushima-shi, Tokushima 770-8501 (JP); UTE, Koichi, Tokushima-shi, Tokushima 770-8501 (JP)
(74) Representative: Duncombe, Jessica
(86) International application number: PCT/JP2021/022105
(87) International publication number: WO 2021/251462

(57) **Abstract**

Provided is a method for simply obtaining a desired size fraction from an aqueous sample containing particles. The method includes the following steps: (1) bringing the aqueous sample containing particles into contact with a superabsorbent polymer to obtain a superabsorbent polymer gel containing a portion of the particles, and (2) mixing the superabsorbent polymer gel with a salt to recover a portion of the particles.

## Description

### Technical Field

The present invention relates to a superabsorbent polymer for classifying particles and a classification method using the superabsorbent polymer.

### Background Art

The condition of an organism is evaluated by taking a biological sample from the organism, extracting the cells or their secretions from the biological sample, and analyzing the proteins, nucleic acids, or other substances contained in the extract.

Cellular secretions include, for example, exosomes, microvesicles, and apoptotic bodies. Of these, exosomes (membrane vesicles with a particle size of 30 to 100 nm) are secreted from a variety of cells, and their presence has been confirmed in every body fluid throughout the body, suggesting that exosomes possibly circulate throughout body fluids to transmit information to remote cells. Substances such as proteins or nucleic acids encapsulated in exosomes reflect in real time the condition of the cells that have secreted them, so exosomes are useful as a biomarker for evaluating, for example, the condition of the original organism or its intercellular signaling.

Size fractionation using an ultracentrifuge is a widely used method in recovering exosomes from a biological sample. This method separates relatively low-density exosomes from other particles by using, for example, a sucrose density gradient.

### Summary of Invention

### Technical Problem

However, size fractionation using an ultracentrifuge is unsatisfactory in terms of the purity of recovered exosomes, and the operation takes a long time.

The first object of the present invention is to provide a method for simply obtaining a desired size fraction from an aqueous sample containing particles (classification method). The second object of the invention is to provide a method for simply separating exosomes from a biological sample.

### Solution to Problem

The present inventors conducted extensive research to achieve the objects and found that a desired size fraction can be simply obtained from an aqueous sample containing particles by bringing the aqueous sample into contact with a superabsorbent polymer to form a gel and mixing the gel with a salt. The inventors conducted further research on the basis of the finding and completed the present invention.

The present invention includes the following aspects.
Item 1. A method for classifying particles A, comprising the following steps:
   (1) bringing an aqueous sample containing the particles A into contact with a superabsorbent polymer to obtain a superabsorbent polymer gel containing a portion of the particles A, and
   (2) mixing the superabsorbent polymer gel with a salt to recover a portion of the particles A.
Item 2. The method according to Item 1, wherein the particles A contain particles A1 with a particle size of 200 nm or less and particles A2 with a particle size of more than 200 nm, and step (2) is a step of recovering the particles A1.
Item 3. A method for separating an exosome from a biological sample, comprising the following steps:
   (3) bringing a biological sample into contact with a superabsorbent polymer to obtain a superabsorbent polymer gel containing the exosome, and
   (4) mixing the superabsorbent polymer gel with a salt to recover the exosome.
Item 4. The method according to Item 3, wherein the biological sample is a body fluid.
Item 5. The method according to Item 3 or 4, wherein the biological sample is urine.
Item 6. The method according to any one of Items 1 to 5, wherein the salt is a metal salt.
Item 7. The method according to any one of Items 1 to 6, wherein the salt is a monovalent or divalent metal salt.
Item 8. The method according to any one of Items 1 to 7, further comprising the step of washing the superabsorbent polymer gel.
Item 9. A method for increasing a cell density in a urine sample, comprising the following steps:
   (5) binging the urine sample into contact with a superabsorbent polymer to obtain a superabsorbent polymer gel, and
   (6) recovering the urine sample that has not been absorbed by the superabsorbent polymer gel.
Item 10. The method according to any one of Items 1 to 9, wherein the superabsorbent polymer is a three-dimensional crosslinked polymer containing a repeating constituent unit represented by the following formula: 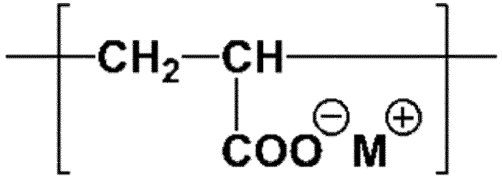 wherein M represents a cation.
Item 11. The method according to any one of Items 1 to 10, wherein the superabsorbent polymer is in a form of particles with an average particle size of 0.1 to 5 mm.
Item 12. A superabsorbent polymer for classifying particles.
Item 13. A superabsorbent polymer for separating an exosome from a biological sample.
Item 14. A superabsorbent polymer for use in the method of any one of Items 1 to 9.
Item 15. The superabsorbent polymer according to any one of Items 12 to 14, which is a three-dimensional crosslinked polymer containing a repeating constituent unit represented by the following formula: 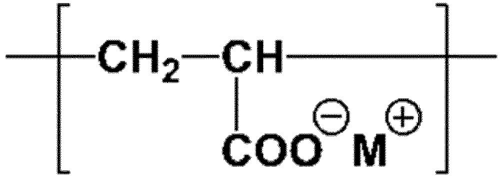 wherein M represents a cation.
Item 16. The superabsorbent polymer according to any one of Items 12 to 15, which is in a form of particles with an average particle size of 0.1 to 5 mm.
Item 17. A kit for classifying particles, comprising a superabsorbent polymer and a salt.
Item 18. A disease test kit using an exosome, comprising a superabsorbent polymer and a salt.
Item 19. The kit according to Item 17 or 18, comprising a three-dimensional crosslinked polymer containing a repeating constituent unit represented by the following formula: 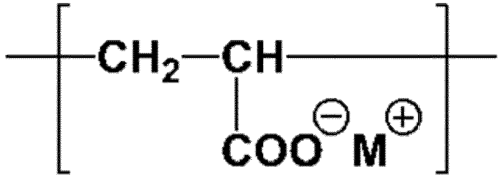 wherein M represents a cation.
Item 20. The kit according to any one of Items 17 to 19, wherein the superabsorbent polymer is in a form of particles with an average particle size of 0.1 to 5 mm.
Item 21. A device for classifying particles, comprising a means for performing the method of Item 1 or 2.
Item 22. A device for separating an exosome from a biological sample, comprising a means for performing the method of any one of Items 3 to 5.

### Advantageous Effects of Invention

The present invention enables obtaining a desired size fraction of particles in a simple manner. The present invention also enables separating cells or their secretions from various biological samples in a simple manner. The separation method according to the present invention increases the purity of a separated substance and decreases the operation time as compared with, for example, conventional ultracentrifugation. The separation method according to the present invention also decreases the number of steps as compared with, for example, conventional ultracentrifugation, and is also excellent in reproducibility. The separated substance is usable in evaluation of the condition of the original organism (e.g., testing or diagnosis). The separated substance is also usable as a carrier for cellularly targeted therapeutic agents.

### Brief Description of Drawings

Fig. 1A: Fig. 1A shows the particle size distribution of latex particles discharged from a superabsorbent polymer (in a 24% aqueous sodium chloride solution).
Fig. 1B: Fig. 1B shows the particle size distribution of original latex particles in a 24% aqueous sodium chloride solution.
Fig. 2A: Fig. 2A shows the particle size distribution of latex particles (in pure water) not absorbed by a superabsorbent polymer.
Fig. 2B: Fig. 2B shows the particle size distribution of original latex particles in pure water.
Fig. 3A: Fig. 3A shows the particle size distribution of latex particles discharged from a superabsorbent polymer (in a 20% aqueous magnesium chloride solution).
Fig. 3B: Fig. 3B shows the particle size distribution of original latex particles in a 20% aqueous magnesium chloride solution.
Fig. 4A: Fig. 4A shows the particle size distribution of latex particles (in pure water) not absorbed by a superabsorbent polymer.
Fig. 4B: Fig. 4B shows the particle size distribution of original latex particles in pure water.
Fig. 5A: Fig. 5A shows the particle size distribution of latex particles discharged from a superabsorbent polymer (in an aqueous solution containing 18% of sodium chloride and 1% of Tween 20).
Fig. 5B: Fig. 5B shows the particle size distribution of original latex particles in an 18% aqueous sodium chloride solution.
Fig. 6: Fig. 6 shows the results of silver staining and western blotting of Example 3, Comparative Example 1, and Comparative Example 2.
Fig. 7: Fig. 7 shows microscopic photographs of an original urine sample and a urine sample obtained in step 4 of Example 3.

### Description of Embodiments

### Particle A Classification Method

In an embodiment, a method for classifying particles A (or substance A to be classified) includes the following steps:
(1) bringing an aqueous sample containing particles A into contact with a superabsorbent polymer to obtain a superabsorbent polymer gel containing a portion of particles A, and
(2) mixing the superabsorbent polymer gel with a salt to recover a portion of particles A.

### Particles A

Particles A are not particularly limited, and may be synthetic particles or biological particles.

Examples of synthetic particles include polymer particles, and specific examples of polymer particles include polyvinyl chloride particles, polymethyl methacrylate particles, polystyrene particles, styrene-butadiene copolymer particles, acrylonitrile-butadiene copolymer particles, methyl methacrylate -butadiene copolymer particles, and silicone particles.

Examples of biological particles include cells (e.g., germ cells, blood cells, fibroblasts, epithelial cells, stem cells, cancer cells, and cultured cells), intracellular organelles (e.g., the nucleus, mitochondria, Golgi apparatus, endoplasmic reticulum, and ribosome), bacteria, viruses, and extracellular vesicles (exosomes, microvesicles, and apoptotic vesicles).

The particle size and average particle size of particles A are each not particularly limited, and may be, for example, 0.3 nm or more, 0.4 nm or more, or 0.5 nm or more, and 10 µm or less, 5 µm or less, or 1 µm or less.

The particle size distribution of particles A may be unimodal or multimodal. In the particle size distribution of particles A, at least one peak may be present within the range of, for example, 30 nm or more, 40 nm or more, or 50 nm or more, and at least one peak may be present within the range of 1000 nm or less, 900 nm or less, 800 nm or less, 700 nm or less, 600 nm or less, 500 nm or less, 400 nm or less, 300 nm or less, or 200 nm or less. The particle size, average particle size, and particle size distribution of particles A can be measured, for example, according to dynamic light scattering.

In an embodiment, particles A preferably contain particles A1 with a particle size of 200 nm or less and particles A2 with a particle size of more than 200 nm. In this embodiment, the "portion of particles A" in step (1) and/or step (2) is preferably particles A1.

The aqueous sample containing particles A may be, for example, latex containing synthetic particles or a biological sample containing biological particles (or a living organism sample). The biological sample may be a biological sample derived from an animal (e.g., a human or a mammal other than humans) or a sample derived from a plant (e.g., an extract). The sample may be a sample derived from a healthy subject or from a patient (e.g., a patient with a lifestyle-related disease, a patient with chronic kidney disease, a neurology patient, a patient with an immune disorder, a cancer patient, a patient with an infection, or a patient with a degenerative disease). Examples of biological samples include body fluids, such as blood, plasma, serum, lachrymal fluid, saliva, breast milk, pleural effusion, peritoneal fluid, amniotic fluid, cerebrospinal fluid, and urine; and liquidized tissue or cells, such as hair, nails, skin, muscles, and nerves. These biological samples may contain exosomes.

The aqueous sample containing particles A may contain any additives. An example of such additives is a surfactant. The surfactant may be an anionic, cationic, nonionic, or ampholytic surfactant, and is preferably a nonionic surfactant. Examples of nonionic surfactants include polyoxyethylene alkyl ethers, such as polyoxyethylene lauryl ether, polyoxyethylene stearyl ether, and polyoxyethylene oleyl ether; polyoxyethylene alkyl phenyl ethers, such as polyoxyethylene octyl phenyl ether and polyoxyethylene nonyl phenyl ether; and polyhydric alcohol fatty acid esters, such as glycerin fatty acid ester, sorbitan fatty acid ester, and pentaerythritol fatty acid ester. These additives may be used singly, or in a combination of two or more.

The concentration of particles A in an aqueous sample is preferably, for example, 1 particle/ml or more, or 2 particles/ml or more, and preferably 50 particles/ml or less, 40 particles/ml or less, or 30 particles/ml or less.

### Superabsorbent Polymer

The superabsorbent polymer (SAP) is a polymer that typically absorbs water in an amount more than 100 times (e.g., 100 to 3000 times, 200 to 2000 times, or 300 to 1000 times) its own weight. In the present invention, although a superabsorbent polymer is preferable for use, any water-absorbing substance can also be similarly used without any limitation. The superabsorbent polymer may be a three-dimensional crosslinked polymer based on a natural polymer (e.g., natural polysaccharides, such as starch, cellulose, alginic acid, chitin, and chitosan), and is preferably a three-dimensional crosslinked polymer containing at least one member selected from the group consisting of a monomer having an acid group (e.g., unsaturated carboxylic acid), a salt thereof, and an amide thereof as a polymerization component.

Examples of the acid group of the monomer having an acid group include a carboxylic acid group (-COOH), a sulfonic acid group [-S(=O)₂(OH)], a sulfuric acid group [-O-S(=O)₂(OH)], a phosphonic acid group [-P(=O) (OH)₂], and a phosphoric acid group [-O-P(=O) (OH)₂]. The monomer may have only one acid group, or two or more acid groups (e.g., a carboxylic acid group and a sulfonic acid group, or a carboxylic acid group and a phosphoric acid group). The salt or amide of a monomer having two or more acid groups may be formed by at least one acid group of them. Typical examples of monomers having an acid group include ethylenically unsaturated monomers having an acid group, such as unsaturated carboxylic acid. The unsaturated carboxylic acid includes, for example, unsaturated monocarboxylic acid and unsaturated dicarboxylic acid. Examples of unsaturated monocarboxylic acid include acrylic acid, methacrylic acid, and crotonic acid. Examples of unsaturated dicarboxylic acid (including its anhydride in the present specification) include maleic acid, fumaric acid, citraconic acid, itaconic acid, and anhydrides of these. The unsaturated carboxylic acid may be used singly, or in a combination of two or more.

The cation of a salt of unsaturated carboxylic acid is not particularly limited and can be, for example, a metal (e.g., a monovalent or divalent metal), or a cation represented by formula NR₄⁺ (wherein each R independently represents a hydrogen atom or a hydrocarbon group optionally having at least one substituent, and two Rs may form a ring together with the adjacent nitrogen atom).

Examples of monovalent metals include alkali metals (e.g., lithium, sodium, and potassium). Examples of divalent metals include alkaline-earth metals (e.g., magnesium, calcium, and barium), lead, zinc, and tin.

In the cation represented by formula NR₄⁺, when R is a hydrocarbon group optionally having at least one substituent, the hydrocarbon group may be, for example, an alkyl group, a cycloalkyl group, an aryl group, or an aralkyl group. Examples of alkyl groups include C₁₋₆ alkyl groups, such as a methyl group, an ethyl group, a propyl group (an n-propyl group or an isopropyl group), a butyl group (an n-butyl group, an isobutyl group, a sec-butyl group, or a t-butyl group). Examples of cycloalkyl groups include C₅₋₁₄ cycloalkyl groups, such as a cyclopentyl group, and a cyclohexyl group. Examples of aryl groups include C₆₋₁₄ aryl groups, such as a phenyl group and a naphthyl group. Examples of aralkyl groups include C₇₋₁₄ aralkyl groups, such as a benzyl group and a phenethyl group.

Examples of optional substituents of the hydrocarbon group include a halogen atom and a hydroxyl group.

When two Rs form a ring together with the adjacent nitrogen atom in the cation represented by formula NR₄⁺, the ring may be a monocyclic ring such as a pyridine ring or an imidazole ring, or a fused ring such as a quinoline ring. The ring may have at least one substituent, and examples of substituents include a halogen atom, a hydroxyl group, an amino group, an alkyl group, a haloalkyl group, a hydroxyalkyl group, and an N,N-dialkylamino group.

The salt of unsaturated carboxylic acid is preferably an alkylamine salt (e.g., a trialkylamine salt, such as a triethyl amine salt), an alkanolamine salt (e.g., a dialkanolamine salt, such as a diethanolamine salt, or a trialkanolamine salt, such as a triethanolamine salt), an ammonium salt, or a tetraalkyl ammonium salt (e.g., a tetramethyl ammonium salt).

The meaning of the amide of unsaturated carboxylic acid includes not only a free amide but also an N-substituted amide (e.g., N-monoalkylamide and N,N-dialkylamide).

The superabsorbent polymer is preferably a three-dimensional crosslinked polymer containing a repeating constituent unit derived from a salt of a monomer having an acid group, more preferably a three-dimensional crosslinked polymer containing a repeating constituent unit derived from a salt of unsaturated carboxylic acid, and still more preferably a three-dimensional crosslinked polymer containing a repeating constituent unit represented by the following formula: 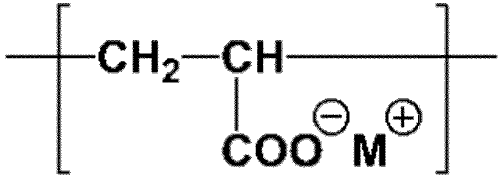 wherein M represents a cation.

The cation represented by M can be the same as those listed as examples for the salt of unsaturated carboxylic acid above.

The superabsorbent polymer may also be a three-dimensional crosslinked polymer containing a repeating constituent unit derived from a monomer having an acid group (preferably a repeating constituent unit derived from unsaturated carboxylic acid). Specifically, the superabsorbent polymer may be a three-dimensional crosslinked polymer containing a monomer having an acid group XH (preferably unsaturated carboxylic acid) as a polymerization component wherein a portion of the acid group XH (preferably COOH) is replaced with the neutralizing base X⁻M⁺ (preferably COO⁻M⁺) of the acid group (e.g., neutralized with a neutralizer (for example, sodium hydroxide when M is sodium)). The degree of neutralization (100 × (the molar number of the neutralizing base of the acid group)/(the total molar number of the acid group and its neutralizing base)) may be, for example, 25 mol% or more, 30 mol% or more, 35 mol% or more, 40 mol% or more, 45 mol% or more, or 50 mol% or more, and 90 mol% or less, 85 mol% or less, or 80 mol% or less.

The superabsorbent polymer may contain as a polymerization component a monomer other than a monomer having an acid group, a salt thereof, and an amide thereof, and may contain an ethylenically unsaturated monomer other than an unsaturated carboxylic acid, a salt thereof, and an amide thereof. Examples of ethylenically unsaturated monomers include the following monofunctional ethylenically unsaturated monomers.
- esters of unsaturated carboxylic acid, such as acrylic acid or methacrylic (e.g., alkyl esters, such as methyl ester, ethyl ester, propyl ester, butyl ester, pentyl ester, hexyl ester, heptyl ester, octyl ester, and 2-ethylhexyl ester; hydroxyalkyl esters, such as 2-hydroxyethyl ester; haloalkyl esters, such as 2,2,2-trifluoroethyl ester; amino alkyl esters, such as 2-amino ethyl ester; (mono- or dialkylamino) alkyl esters, such as 2-(N,N-dimethylamino) ethyl ester; cycloalkyl esters, such as cyclohexyl ester; aryl esters, such as phenyl ester and naphthyl ester; aralkyl esters, such as benzyl ester and phenethyl ester; glycidyl ester; and polyethylene glycol ester)
- unsaturated dicarboxylic imide (e.g., maleimide, citraconimide, itaconimide, N-alkyl-substituted form of these, N-cycloalkyl substituted form of these, or N-aryl substituted form of these)
- alkenyl esters of saturated carboxylic acid, such as acetic acid or propionic acid (e.g., vinyl ester and allyl ester)
- unsaturated sulfonic acid (e.g., vinyl sulfonic acid and allyl sulfonic acid), a salt thereof, an ester thereof, or an amide thereof
- unsaturated alcohol (e.g., allyl alcohol and propenyl alcohol)
- unsaturated ether (e.g., alkyl vinyl ethers, such as methyl vinyl ether and ethyl vinyl ether; alkyl allyl ethers, such as methyl allyl ether and ethyl allyl ether; cycloalkyl vinyl ethers, such as cyclohexyl vinyl ether; and glycidyl vinyl ether)
- unsaturated nitrile (e.g., acrylonitrile and methacrylonitrile)
- olefins (e.g., ethylene, propylene, butene, pentene, and hexene)
- aromatic vinyl compounds (e.g., styrene, α-methylstyrene, vinyltoluene, and hydroxystyrene)
- heterocyclic vinyl compounds (e.g., N-vinylpyrrolidone)
These may be used singly, or in a combination of two or more.

The superabsorbent polymer is preferably a three-dimensional crosslinked polymer three-dimensionally crosslinked with a crosslinking agent. Examples of crosslinking agents include, but are not particularly limited to, the following difunctional or higher functional ethylenically unsaturated monomers.
- alkenyl ethers of dihydric or higher alcohol (e.g., alkylene glycols, such as ethylene glycol, polyethylene glycol, propylene glycol, butylene glycol, or neopentyl glycol, polyalkylene glycol, glycerin, polyglycerin, trimethylol ethane, trimethylol propane, pentaerythritol, dipentaerythritol, and sorbitan) (e.g., vinyl ether and allyl ether)
- esters of unsaturated carboxylic acid (e.g., acrylic acid and methacrylic acid) and dihydric or higher alcohol (e.g., those listed as examples for ethers above)
- alkenyl esters of unsaturated carboxylic acid (e.g., acrylic acid and methacrylic acid) (e.g., vinyl ester and allyl ester)
- alkenyl esters of polycarboxylic acid (e.g., tartaric acid, citric acid, and adipic acid) (e.g., vinyl ester and allyl ester)
- alkenyl esters of isocyanuric acid (e.g., triallyl isocyanate)
- alkylene bis acrylamide (e.g., methylene bis acrylamide)
- alkylene bis methacrylamide (e.g., methylene bis methacrylamide)
- di- or trialkenyl amines (e.g., diallyl amine and triallyl amine)
- aromatic polyvinyl compounds (e.g., divinyl benzene)
These may be used singly, or in a combination of two or more.

The crosslinking agent for use can be a crosslinking agent having at least two functional groups reactive with a monomer having an acid group, a salt thereof, or an amide thereof. Examples of such crosslinking agents include polyhydric alcohol glycidyl ethers, and specific examples include alkylene glycol diglycidyl ethers, such as ethylene glycol diglycidyl ether; and alkane triol diglycidyl ethers or alkane triol triglycidyl ethers, such as glycerin diglycidyl ether or glycerin triglycidyl ether.

The amount of the crosslinking agent can be suitably selected according to the desired crosslink density, and can be, for example, 0.005 mol% or more, or 0.01 mol% or more, and 0.5 mol% or less, or 0.4 mol% or less, based on the polymerization components (the total of unsaturated carboxylic acid, a salt thereof, an amide thereof, and any monofunctional ethylenically unsaturated monomers other than these).

The superabsorbent polymer is preferably in the form of particles with an average particle size (e.g., volume average particle size) within the range of 50 µm or more, 60 um or more, 70 um or more, 80 um or more, 90 um or more, 0.1 mm or more, 0.2 mm or more, 0.3 mm or more, 0.4 mm or more, 0.5 mm or more, or 1 mm or more, and preferably particles with an average particle size (e.g., volume average particle size) within the range of 5 mm or less, 4 mm or less, 3 mm or less, 2 mm or less, or 1 mm or less. The average particle size can be measured, for example, according to dynamic light scattering or image analysis using a commonly used device (e.g., Camsizer (trademark), Retsch GmbH).

The superabsorbent polymer can be produced, for example, by a method including the step of polymerizing a composition containing a polymerization component, a solvent, and an initiator (e.g., solution polymerization, emulsion polymerization, or suspension polymerization), the step of drying the polymerized product, and the step of optionally classifying the polymerized product. The superabsorbent polymer for use may be a commercial product.

The amount of the superabsorbent polymer for use is preferably, for example, 1 part by mass or more, 5 parts by mass or more, or 10 parts by mass or more, and preferably 2000 parts by mass or less, 1500 parts by mass or less, or 1000 parts by mass or less, per 100 parts by mass of particles A.

The temperature at which the aqueous sample is brought into contact with the superabsorbent polymer is preferably, for example, 1 to 35°C, or 5 to 30°C. After contact, it is preferable to allow the aqueous sample to stand for a predetermined time (e.g., 1 hour or more or 2 hours or more, or 5 hours or less or 4 hours or less).

Due to step (1), particles A can be sieved and separated according to their size into those inside the superabsorbent polymer gel and those outside the superabsorbent polymer gel. For example, in the case of a biological sample, separation can be made such that extracellular vesicles (e.g., exosomes) are present inside the superabsorbent polymer gel, and cells are present outside the superabsorbent polymer gel.

The method for classifying particles A preferably further includes the step of washing the superabsorbent polymer gel. The step is preferably performed after step (1) and before step (2). The recovery rate of particles can be further increased by including this step. Although the washing solution may be pure water, the washing solution is preferably water containing a surfactant, and particularly preferably deionized water containing a surfactant. The surfactant for use may be, for example, the same as the surfactant that can be contained in the aqueous sample containing particles A, and is preferably a nonionic surfactant. The content of a surfactant in water containing the surfactant can be, for example, 0.1 parts by mass or more, 0.5 parts by mass or more, or 1 part by mass or more, and 1 part by mass or less, 2 parts by mass or less, 3 parts by mass or less, 4 parts by mass or less, or 5 parts by mass or less, per 100 parts by mass of water.

### Salt

The salt is not particularly limited as long as the salt can discharge the particles contained in the superabsorbent polymer gel. Examples of salts include metal salts (e.g., a monovalent or divalent metal salt).

Examples of monovalent metal salts include alkali metal salts (e.g., sodium salts, and potassium salts). Examples of divalent metal salts include alkaline-earth metal salts (e.g., magnesium salts, calcium salts, and barium salts).

The counter anion of the salt can be, for example, but is not limited to, a halide ion, such as a chloride ion or a bromide ion.

The salt may be used singly, or in a combination of two or more.

The salt is preferably at least one member selected from the group consisting of sodium chloride and magnesium chloride. The salt may be used in the form of an aqueous solution.

The amount of the salt can be, for example, 1 part by mass or more, 5 parts by mass or more, or 10 parts by mass or more, and 2000 parts by mass or less, 1500 parts by mass or less, or 1000 parts by mass or less, per 100 parts by mass of the superabsorbent polymer gel.

The temperature at which the superabsorbent polymer gel is mixed with the salt is preferably, for example, 1 to 35°C, or 5 to 30°C. After being mixed, the mixture is preferably allowed to stand for a predetermined time (e.g., 1 hour or more or 2 hours or more, or 5 hours or less or 4 hours or less).

Due to step (2), the particles contained in the superabsorbent polymer gel (some or all of the particles) can be discharged (or recovered). The classification method according to the present invention enables recovery of particles at a high recovery rate.

In an embodiment, the method for classifying particles A is preferably a method for separating (or purifying) exosomes from biological particles, and preferably includes the following steps:
(3) bringing a biological sample into contact with a superabsorbent polymer to obtain a superabsorbent polymer gel containing exosomes, and
(4) mixing the superabsorbent polymer gel with a salt to recover the exosomes.

In this embodiment, the type and the amount of the biological sample, superabsorbent polymer, and salt for use can be as described above. The biological sample is preferably a body fluid, and more preferably urine.

In this embodiment, the mixture of the superabsorbent polymer gel and the salt may be dialyzed.

In this embodiment, the purity of recovered exosomes can be increased (the amount of protein in urine as foreign matter being decreased), and the operation time can be shortened, compared with conventional ultracentrifugation.

### Method for Increasing the Cell Density in a Urine Sample

The present invention includes a method for increasing the cell density in a urine sample, and the method includes the following steps:
(5) bringing a urine sample into contact with a superabsorbent polymer to obtain a superabsorbent polymer gel, and
(6) recovering the urine sample that has not been absorbed by the superabsorbent polymer gel.

The superabsorbent polymer and the amount of the superabsorbent polymer for use may be the same as those described as examples in the "Particle A Classification Method" section above.

The recovered urine sample contains cells (e.g., red blood cells, white blood cells, epithelial cells (e.g., cells shed from the kidney, renal tubular, or bladder), and urinary casts containing these cells) at a higher density than in the original urine sample, and can be suitably used in testing for various diseases.

In this method, the conventional step of centrifuging a urine sample can be omitted.

### Superabsorbent Polymer for Classifying Particles

The present invention includes a superabsorbent polymer for classifying particles. The particles and the superabsorbent polymer can be respectively the same as particles A and the superabsorbent polymer described in the "Particle A Classification Method" section above.

The superabsorbent polymer for classifying particles is preferably a superabsorbent polymer for use in the method for classifying particles A described above, and is more preferably a superabsorbent polymer for separating exosomes from a biological sample.

### Kit for Classifying Particles

The present invention includes a kit for classifying particles, including a superabsorbent polymer and a salt. The particles, superabsorbent polymer, and salt can be respectively the same as particles A, the superabsorbent polymer, and the salt described in the "Particle A Classification Method" section above. The kit may further contain a washing solution. The washing solution can be the same as the washing solution described in the step of washing the superabsorbent polymer gel. The kit may further contain an instruction manual on the method for classifying particles.

### Disease Test Kit Using Exosomes

The present invention includes a disease test (or disease diagnosis) kit using exosomes, containing a superabsorbent polymer and a salt.

The test subject may be a healthy subject or a patient. Examples of diseases include lifestyle-related diseases, chronic kidney disease, neurological diseases, immune disorders, cancers, infections, and degenerative diseases. Exosomes can be collected from, for example, a body fluid of the test subject (e.g., blood, plasma, serum, lachrymal fluid, saliva, breast milk, pleural effusion, peritoneal fluid, amniotic fluid, cerebrospinal fluid, or urine), or liquidized cells or tissue. The kit may be a kit modified to contain a superabsorbent polymer and a salt as reagents for collecting exosomes from a test subject in a known disease test kit using exosomes.

The superabsorbent polymer and the salt can be respectively the same as the superabsorbent polymer and the salt described in the "Particle A Classification Method" section above. The kit may further contain a washing solution. The washing solution can be the same as the washing solution described in the step of washing the superabsorbent polymer gel. The kit may further contain an instruction manual on the method for collecting exosomes from a test subject.

### Device for Classifying Particles

The present invention includes a device for classifying particles, including a means for performing the method for classifying particles A described above.

### Device for Separating Exosomes from a Biological Sample

The present invention includes a device for separating exosomes from a biological sample, including a means for performing the method for separating exosomes from a biological sample.

### Examples

The following describes the present invention in detail with reference to Examples. However, the present invention is not limited to these Examples.

### Example 1

To demonstrate the size-sorting effect of a superabsorbent polymer in the absorption-discharge process for nanoparticles, the following experiment was performed.

Deionized water containing 0.20% Tween 20 (Rheodol TW-L120, Kao Chemicals, a nonionic surfactant) was individually added to polystyrene latex with an average particle size of 100 nm (LB1, Sigma-Aldrich) and polystyrene latex with an average particle size of 300 nm (LB3, Sigma-Aldrich). Each mixture was diluted by a factor of 1000, and then both mixtures were mixed in equal amounts. This mixture is referred to as a "latex mixture solution" below.

Four commercially available superabsorbent polymer beads (60 mg) used in home hydroponics *(Vixker puyo puyo ball,* a crosslinked polymer containing sodium polyacrylate as a main component, spherical, particle size: 2.66 ± 0.11 mm, particle size distribution: normal distribution) were placed in a glass sample tube (volume: 50 mL), and then 5.868 g of the latex mixture solution [a] was added to the tube, followed by allowing the mixture to stand at room temperature for 3 hours. 2.170 g of the solution that was not absorbed by the polymer beads [b] was removed, and 1.003 g of sodium chloride [c] was added, followed by stirring with a vortex mixer for 10 seconds. The mixture was allowed to stand at room temperature for 2 hours, and 2.909 g of the solution discharged from the polymer beads [d] was recovered. The recovery rate [100 × d/(a - b + c)] was 61.9%.

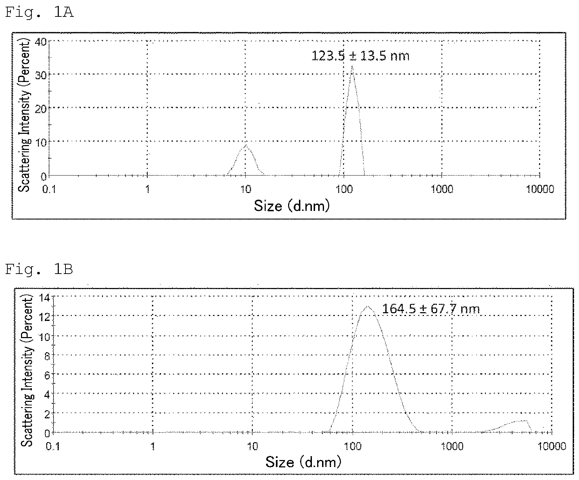

Fig. 1A shows the results of measuring the particle size distribution of the polystyrene latex contained in the collected solution according to dynamic light scattering. Measurement was performed with a ZetaSizer Nano ZSP device (Malvern) and a 10-mm square glass cell, and analysis was performed at a measurement temperature of 30°C, a solvent refractive index of 1.367, and a solvent viscosity of 1.394 cP (which corresponds to a 24% aqueous sodium chloride solution) according to the non-negative least square method. For comparison, a 24% solution of sodium chloride in the original latex mixture solution was also measured for particle size distribution under the same conditions (Fig. 1B). Whereas the average particle size of the particles of the original latex mixture solution was 164.5 ± 67.7 nm, the average particle size of the particles in the effluent was 123.5 ± 13.5 nm. This indicates that smaller particles (with a particle size of 137 nm or less) were sorted and recovered in the process of discharging particles from the polymer beads.

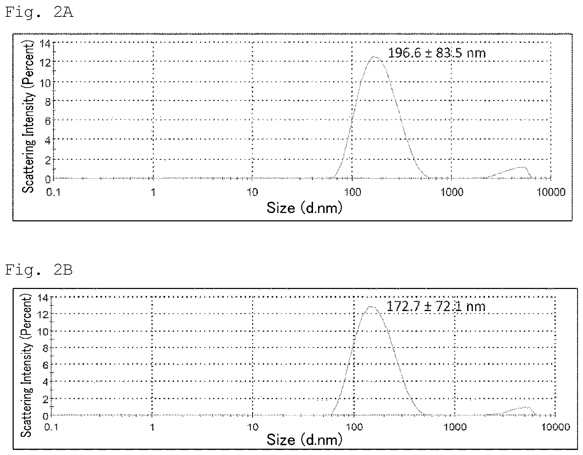

Whereas the average particle size of the particles that were not absorbed by the polymer beads and that remained in mixture solution (b) was 196.6 ± 83.5 nm (Fig. 2A), the average particle size of the particles in the original latex mixture solution was 172.7 ± 72.0 nm (Fig. 2B). This suggests that size classification also occurs in the process of absorbing the particles by the polymer beads. The particle size distribution shown in Fig. 2 was analyzed by using the refractive index (1.330) and the viscosity (0.792 cP) of pure water.

### Example 2A

### Use of Aqueous Magnesium Chloride Solution in Operation of Discharging Nanoparticles (Alternative Method of Example 1).

The operation of discharging nanoparticles from a superabsorbent polymer gel can also be performed by using an aqueous magnesium chloride solution instead of sodium chloride powder (Example 1). The chelating effect between the carboxyl groups and divalent magnesium ions in a superabsorbent polymer increases the degree of contraction of the polymer gel, allowing nanoparticles to be discharged more efficiently.

Deionized water containing 0.20% Tween 20 was added to polystyrene latex with an average particle size of 100 nm and polystyrene latex with an average particle size of 600 nm (LB6, Sigma-Aldrich), and each mixture was diluted by a factor of 1000, followed by mixing both in equivalent amounts. This mixture is referred to as a "latex mixture solution B" below.

Four superabsorbent polymer beads (60 mg) were placed in a glass sample tube (volume: 50 mL), and then 5.864 g of latex mixture solution B [a] was added to the tube, followed by allowing the mixture to stand at room temperature for 3 hours. 2.267 g of the remaining solution that was not absorbed by the polymer beads [b] was removed, and 4.738 g of a 54% aqueous magnesium chloride solution [c] was added, followed by stirring with a vortex mixer for 10 seconds. The mixture was allowed to stand at room temperature for 2 hours, and 7.254 g of the solution discharged from the polymer beads [d] was recovered. The recovery rate [100 × d/(a - b + c)] was 87.0%.

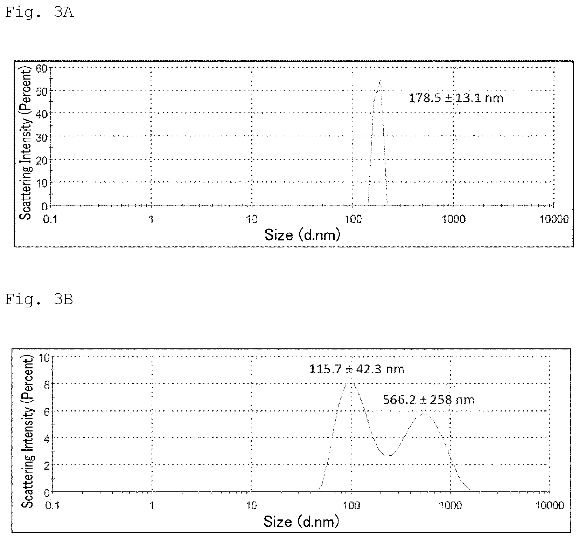

Fig. 3A shows the particle size distribution of the polystyrene latex contained in the collected solution. Measurement according to dynamic light scattering was performed with the same device as in Example 1, and analysis was performed at a measurement temperature of 30°C, a solvent refractive index of 1.376, and a solvent viscosity of 2.170 cP (which corresponds to a 20% aqueous magnesium chloride solution). For comparison, a solution prepared by mixing original latex mixture solution B with a 54% aqueous magnesium chloride solution (which corresponds to a 20% aqueous magnesium chloride solution) was also measured for particle size distribution under the same conditions (Fig. 3B). Whereas the particle size of the particles in latex mixture solution B showed bimodal distribution of 115.7 ± 42.3 nm and 566.2 ± 258 nm (Fig. 3B), the particle size distribution of the particles in the effluent was unimodal, with an average particle size of 178.5 ± 13.1 nm (Fig. 3A). This indicates that smaller particles (particle size: 192 nm or less) were sorted and recovered in the process of discharging particles from the polymer beads.

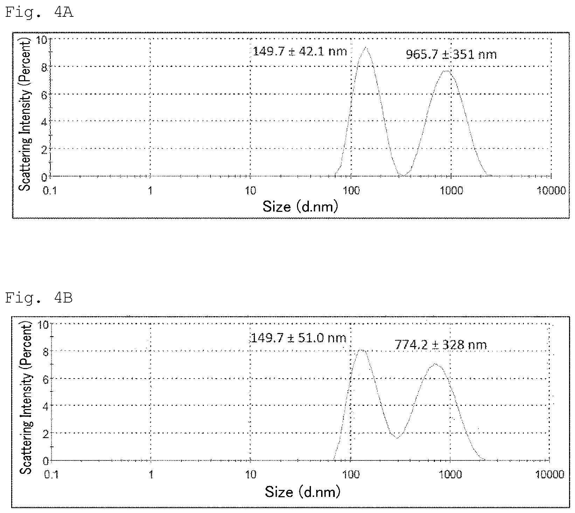

Whereas the particle size of the particles that were not absorbed by the polymer beads and that remained in mixture solution (b) showed bimodal distribution of 149.7 ± 42.1 nm and 965.7 ± 351 nm (Fig. 4A), the particle size of the particles in latex mixture solution B showed bimodal distribution of 149.7 ± 51.0 nm and 774.2 ± 328 nm (Fig. 4B). A comparison of latex particle size between these two solutions indicates that while there is no difference in average particle size of smaller particles, the average particle size of large particles (965.7 nm) of the former was larger than that of the latter (774.2 nm), suggesting that size classification occurs even in the process of absorbing the particles by the polymer beads. The particle size distribution shown in Fig. 4 was analyzed by using the values of the refractive index and viscosity of pure water.

### Example 2B

### Washing with Deionized Water before the Operation of Discharging Nanoparticles (Alternative Method of Example 1).

Deionized water containing 0.20% Tween 20 was added to polystyrene latex with an average particle size of 100 nm (LB1, Sigma-Aldrich) and polystyrene latex with an average particle size of 600 nm (LB6, Sigma-Aldrich), and each mixture was diluted by a factor of 1000, followed by mixing both mixtures in equal amounts. This mixture is referred to as "latex mixture solution B" below.

Four superabsorbent polymer beads (60 mg) were placed in a glass sample tube (volume: 50 mL), and then 5.970 g of latex mixture solution B [a] was added to the tube, followed by allowing the mixture to stand at room temperature for 3 hours. 1.961 g of the solution that was not absorbed by the polymer beads [b] was removed, and the polymer bead surface was washed with 1 mL of deionized water containing 1% Tween 20 three times. Subsequently, 8.077 g of an about 26% aqueous sodium chloride solution [c] was added to the polymer beads and stirred with a vortex mixer for 10 seconds. After stirring at room temperature for 2 hours, 10.795 g of the solution discharged from the polymer beads [d] was recovered. The recovery rate [100 × d/(a - b + c)] was 89%.

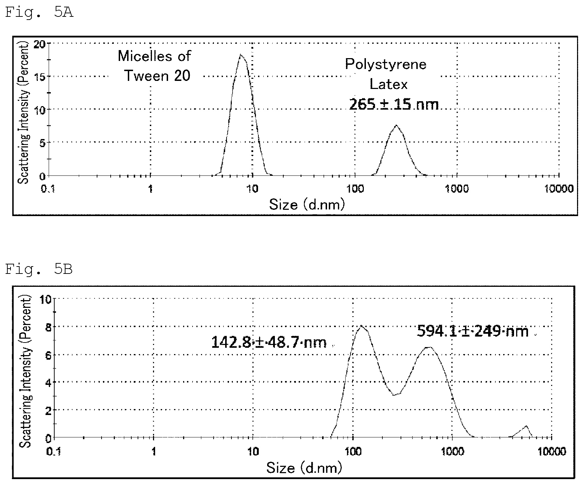

To prevent the aggregation of the latex particles, 1% of Tween 20 was added to the collected solution. Fig. 5A shows the particle size distribution of the polystyrene latex contained in the recovered solution. Measurement according to dynamic light scattering (DLS) was performed with the same device as in Example 1, and analysis was performed at a measurement temperature of 30°C, a solvent refractive index of 1.358, and a solvent viscosity of 1.1723 cP (which corresponds to an 18% aqueous sodium chloride solution) according to the non-negative least square method. For comparison, a solution prepared by mixing the original latex mixture solution B with an aqueous sodium chloride solution (which corresponds to an 18% aqueous sodium chloride solution) was also measured for particle size distribution under the same conditions (Fig. 5B). Whereas the particle size distribution of the particles in the original latex mixture solution B showed bimodal distribution (peak top particle size: 142.8 nm and 594.1 nm), the particle size distribution of the particles in the effluent was unimodal, with a peak top particle size of 264.5 nm (the average of three measurements by DLS). In the particle size distribution shown in Fig. 5A, a peak of micelles formed by Tween 20 added for prevention of aggregation (average particle size: 8.1 nm) was also observed.

### Example 3

The following steps were performed in sequence to recover exosomes from a urine sample.
1. Superabsorbent polymer beads *(Vixker puyo puyo ball,* a crosslinked polymer containing sodium polyacrylate as a main component, spherical, particle size: 2.66 ± 0.11 mm, particle size distribution: normal distribution) were placed in a 100-mL urine collection cup to give a surface area of 100 mm².
2. 7 ml of a urine sample was added to the urine collection cup containing the superabsorbent polymer beads.
3. The urine sample in the cup was allowed to stand at room temperature for 3 hours.
4. The urine sample that was not absorbed by the superabsorbent polymer gel was collected.
5. The surface of the superabsorbent polymer gel was washed with 1 mL of pure water three times.
6. The superabsorbent polymer gel was collected in a centrifuge tube, and NaCl in 1/10 the volume of the urine sample was added.
7. The mixture was vortexed for 10 seconds and allowed to stand at room temperature for about 1 to 2 hours.
8. The solution discharged from the superabsorbent polymer gel was collected.
9. The collected solution was placed in a dialysis membrane (SpectrumLabs.com, Spectra/Por, MWCO: 3500), and dialyzed against PBS (2 hours, room temperature).
10. The concentration of protein in the dialyzed solution was measured according to the Bradford assay.
11. Silver staining and western blotting were performed.

The silver staining in step 11 was performed according to the following procedure.

2 to 15 µL of an exosome-derived protein sample was subjected to electrophoresis (SDS-PAGE) using a 10% polyacrylamide gel, and then subjected to silver staining with a silver staining kit (trade name: EzStain Silver, ATTO).

The western blotting in step 11 was performed according to the following procedure.

### Electrophoresis (SDS-PAGE)

2 to 15 µL of an exosome-derived protein sample was subjected to electrophoresis (SDS-PAGE) using a 10% polyacrylamide gel.

### Blotting

Extra Thick Blot Paper (Bio-Rad) and a Hybond ECL nitrocellulose membrane (GE Healthcare) were individually immersed in Milli-q water, and then immersed in a transfer buffer (tris: 3.03 g, glycine: 14.41 g, SDS: 0.1 g, methanol: 200 mL, Milli-Q water: enough to make 1 L) for 20 to 40 minutes. The polyacrylamide gel after electrophoresis was also immersed in a transfer buffer for 20 minutes. From these, a laminate of Extra Thick Blot Paper, Hybond ECL nitrocellulose membrane, polyacrylamide gel, and Extra Thick Blot Paper was prepared, and placed in a platinum anode in the lower part of a blotting device. The bubbles between layers were removed, and a stainless-steel cathode and a safety cover in the upper part were attached, followed by performing blotting.

### Washing the Membrane

The transferred membrane was washed with 20 mL of autoclaved Milli-Q water for 5 minutes twice and then immersed in 10 mL of a blocking solution, followed by incubation on an orbital shaker for 60 minutes. The membrane was then rinsed with 20 mL of TBS-T (1 mL of Tween 20 and 1 L of TBS (8 g of NaCl, 20 mL of Tris-HCl (pH: 7.6), enough Milli-Q water to make 1 L)) for 5 minutes. This rinsing operation was repeated at least one time.

### Primary Antibody Reaction

The washed membrane was immersed in 10 mL of a primary antibody solution (about 0.2 to 1 µg/mL; anti-CD9-antibody, anti-CD63-antibody, or anti-CD81-antibody) and then incubated at room temperature for at least 1 hour. The membrane was rinsed with 20 mL of TBS-T for 5 minutes, and this rinsing operation was repeated at least one time. After washing with 200 mL of TBS-T for 15 minutes, the membrane was rinsed again with 20 mL of TBS-T for 5 minutes, and this rinsing operation was repeated at least two times.

### Secondary Antibody Reaction

After being subjected to a primary antibody reaction, the membrane was immersed in 10 mL of an HRP-labeled secondary antibody solution and then incubated on an orbital shaker at room temperature for 60 minutes. The membrane was rinsed with 20 mL of TBS-T for 5 minutes, and this rising operation was repeated at least two times. After washing with 200 mL of TBS-T for 15 minutes, the membrane was rinsed again with 20 mL of TBS-T for 5 minutes, and this rinsing operation was repeated at least two times.

### Detection

After being subjected to a secondary antibody reaction, the membrane was immersed in a detection solution (1.5 mL of ECL Advance Solution A, 1.5 mL of ECL Advance Solution B, and 1.5 mL of autoclaved Milli-Q water) and allowed to undergo a reaction on a seesaw shaker for 5 minutes. The membrane was placed on a transparent plastic sheet with the protein side up and covered with another transparent plastic sheet, followed by irradiation with X-rays for 15 seconds to 1 hour.

### Comparative Example 1

Exosomes were recovered from a urine sample by ultracentrifugation, and silver staining and western blotting were performed in the same manner as in Example 3.

### Comparative Example 2

Exosomes were recovered from a urine sample by using a commercially available kit (Exo-Urine^{™} EV Isolation Kit, SBI), and silver staining and western blotting were performed in the same manner as in Example 3.

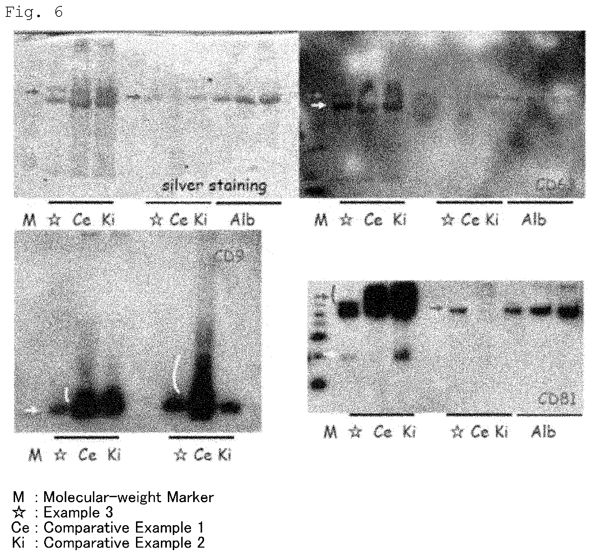

Fig. 6 shows the results of silver staining and western blotting in Example 3 and Comparative Examples 1 and 2. As is clear from Fig. 6, exosomes were recovered with higher purity in Example 3 than in Comparative Examples 1 and 2.

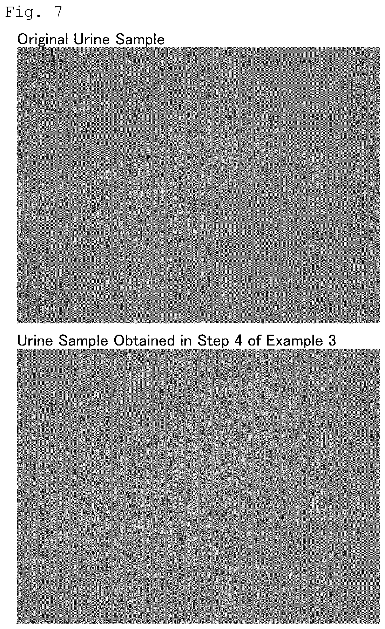

Fig. 7 shows the microscopic photographs of the original urine sample and the urine sample obtained in step 4 of Example 3. As is clear from Fig. 7, the urine sample obtained in step 4 of Example 3 had a higher cell density than the original urine sample and is thus useful in testing for various diseases.

## Claims

1. A method for classifying particles A, comprising the following steps:
(1) bringing an aqueous sample containing the particles A into contact with a superabsorbent polymer to obtain a superabsorbent polymer gel containing a portion of the particles A, and
(2) mixing the superabsorbent polymer gel with a salt to recover a portion of the particles A.

2. The method according to claim 1, wherein the particles A contain particles A1 with a particle size of 200 nm or less and particles A2 with a particle size of more than 200 nm, and step (2) is a step of recovering the particles A1.

3. A method for separating an exosome from a biological sample, comprising the following steps:
(3) bringing a biological sample into contact with a superabsorbent polymer to obtain a superabsorbent polymer gel containing the exosome, and
(4) mixing the superabsorbent polymer gel with a salt to recover the exosome.

4. The method according to claim 3, wherein the biological sample is a body fluid.

5. The method according to claim 3 or 4, wherein the biological sample is urine.

6. The method according to any one of claims 1 to 5, wherein the salt is a metal salt.

7. The method according to any one of claims 1 to 6, wherein the salt is a monovalent or divalent metal salt.

8. The method according to any one of claims 1 to 7, further comprising the step of washing the superabsorbent polymer gel.

9. A method for increasing a cell density in a urine sample, comprising the following steps:
(5) binging the urine sample into contact with a superabsorbent polymer to obtain a superabsorbent polymer gel, and
(6) recovering the urine sample that has not been absorbed by the superabsorbent polymer gel.

10. The method according to any one of claims 1 to 9, wherein the superabsorbent polymer is a three-dimensional crosslinked polymer containing a repeating constituent unit represented by the following formula: 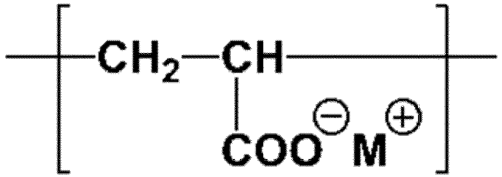 wherein M represents a cation.

11. The method according to any one of claims 1 to 10, wherein the superabsorbent polymer is in a form of particles with an average particle size of 0.1 to 5 mm.

12. A superabsorbent polymer for classifying particles.

13. A superabsorbent polymer for separating an exosome from a biological sample.

14. A superabsorbent polymer for use in the method of any one of claims 1 to 9.

15. The superabsorbent polymer according to any one of claims 12 to 14, which is a three-dimensional crosslinked polymer containing a repeating constituent unit represented by the following formula: 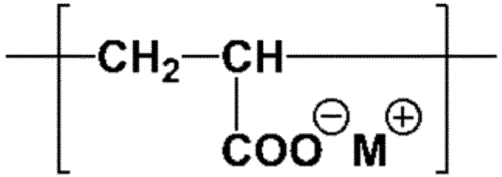 wherein M represents a cation.

16. The superabsorbent polymer according to any one of claims 12 to 15, which is in a form of particles with an average particle size of 0.1 to 5 mm.

17. A kit for classifying particles, comprising a superabsorbent polymer and a salt.

18. A disease test kit using an exosome, comprising a superabsorbent polymer and a salt.

19. The kit according to claim 17 or 18, comprising a three-dimensional crosslinked polymer containing a repeating constituent unit represented by the following formula: 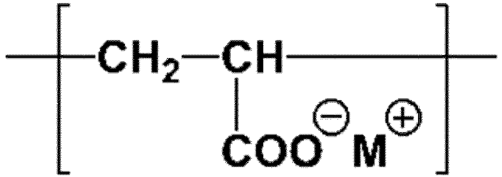 wherein M represents a cation.

20. The kit according to any one of claims 17 to 19, wherein the superabsorbent polymer is in a form of particles with an average particle size of 0.1 to 5 mm.

21. A device for classifying particles, comprising a means for performing the method of claim 1 or 2.

22. A device for separating an exosome from a biological sample, comprising a means for performing the method of any one of claims 3 to 5.
